(19) Europäisches Patentamt
European Patent Office
Office européen des brevets

(11) EP 0 859 003 A2

# (12) EUROPÄISCHE PATENTANMELDUNG

(43) Veröffentlichungstag:
**19.08.1998 Patentblatt 1998/34**

(51) Int. Cl.[6]: **C07D 327/02**, A61K 7/46

(21) Anmeldenummer: **98102035.7**

(22) Anmeldetag: **06.02.1998**

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(30) Priorität: **14.02.1997 EP 97102395**

(71) Anmelder:
**GIVAUDAN-ROURE (INTERNATIONAL) S.A.
1214 Vernier, Genève (CH)**

(72) Erfinder:
- **Bajgrowicz, Jerzy A.
8053 Zürich (CH)**
- **Frater, Georg
8400 Winterthur (CH)**
- **Kraft, Philip
8600 Dübendorf (CH)**

(74) Vertreter: **Buntz, Gerhard et al
F.Hoffmann-La Roche AG
Patent Department (PLP),
124 Grenzacherstrasse
4070 Basel (CH)**

## (54) Thiamakrolide

(57) Die Erfindung betrifft neue Thiamakrolide, insbesondere Moschusriechstoffe, mit extrem niedrigen Schwellenwerten, ihre Verwendung in der funktionellen Parfümerie wie der Feinparfümerie, sowie ein Verfahren zur Herstellung dieser und analoger Verbindungen.

EP 0 859 003 A2

## Beschreibung

Die Erfindung betrifft neue Thiamakrolide, insbesondere Moschusriechstoffe, mit extrem niedrigen Schwellenwerten, ihre Verwendung in der funktionellen Parfümerie wie der Feinparfümerie, sowie ein Verfahren zur Herstellung dieser und analoger Verbindungen.

Wegen ihres *warmen, erogen-animalischen und langanhaftenden* Geruches zählen Moschusriechstoffe zu den in der Parfümerie am meisten verwendeten Verbindungen. Die schlechte biologische Abbaubarkeit, eine gewisse Phototoxizität sowie die vermutete Neurotoxizität benzoider MoschusRiechstoffe hat in letzter Zeit zum vermehrten Einsatz makrocyclischer Verbindungen geführt, die sich von natürlich vorkommenden Moschuskörpern ableiten und ökologisch unbedenklich sind. Hohe Produktionskosten verhindern jedoch noch ihren Einsatz in größerem Maßstab. Einen Ausweg würden Makrocyclen bieten, die durch ihren niedrigen Schwellenwert in geringeren Konzentrationen als üblich und damit zu niedrigeren Kosten eingesetzt werden könnten. Obwohl man sich an Leitstrukturen orientieren kann und trotz zahlreicher Untersuchungen über Struktur-Geruch-Beziehungen von Riechstoffen [P. Weyerstahl, *J. prakt. Chem.* **1994**, *336,* 95; K. J. Rossiter, *Chem. Rev.* **1996**, *96,* 3240] ist eine Vorhersage der Intensität oder des Schwellenwertes von Makrocyclen mit Moschusgeruch bislang unmöglich.

Wir fanden nun eine kleine Klasse von Thiamakroliden **I**, die extrem niedrige Schwellenwerte, nämlich von 0.1-0.3 ng/l Luft besitzen, d. h. bis zu zehnmal niedriger als der Schwellenwert des am häufigsten verwendeten Makrolids 15-Pentadecanolid (**5**, 1.1 ng/l). Dies ist umso erstaunlicher, als Thiamakrolide bereits Gegenstand einer Erfindung waren [Naarden, Niederl. Patent No. 96391], sich die dort synthetisierten Verbindungen im Geruch jedoch als sehr viel schwächer als 15-Pentadecanolid (**5**) herausgestellt haben [E. T. Theimer, J. T. Davies, *J. Agr. Food Chem.* **1967**, *15,* 8].

Diese neue Klasse von Thiamakroliden kann durch die allgemeine Formel

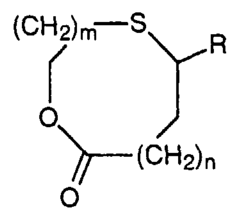

**I**

worin R=H und worin
m=9 und n=3, oder
m=7 und n=4, oder
m=3 und n=8, oder
m=7 und n=3 dargestellt werden.

Sie umfasst demgemäss die Verbindungen der Formeln **1-4.**

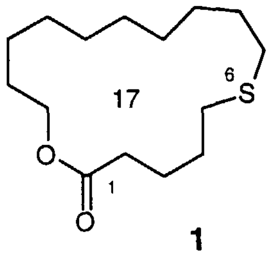

**1**

0.1 ng/l

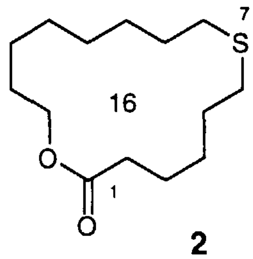

**2**

0.2 ng/l

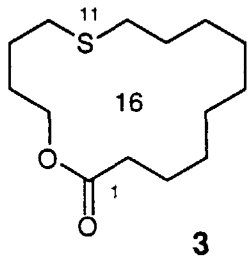

**3**

0.2 ng/l

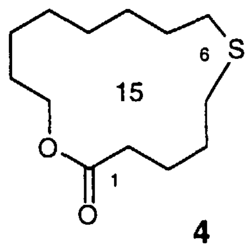

**4**

0.3 ng/l

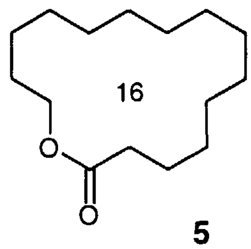

**5**

1.1 ng/l

Neben extrem niedrigen Schwellenwerten besitzen die Verbindungen **I** eine sehr gute Haftfestigkeit. Alle weisen *intensive Moschus-Noten auf, die oft von pudrig-süssen Akzenten begleitet* werden. Anders als entsprechende Verbindungen ohne Schwefelatome und analoge ungesättigte Makrolide, zeichnen sich diese Verbindungen meistens auch durch *warme, grün-moosige Nuancen* aus. Einen besonders schönen parfümistischen Effekt gibt dieses Zusammenspiel *pudrig-süsser und grün-moosiger Elemente bei dominanter Moschusnote* bei der Verbindung **2**.

Die Thiamakrolide **I** lassen sich generell wie die bekannten Moschusriechstoffe einsetzen, sie harmonieren also mit einer Vielzahl natürlicher wie synthetischer Produkte, die häufig in Riechstoffkompositionen eingesetzt werden. Insbesondere in der Basisnote erzielen sie in Kombination mit holzigen und ambrierten Akkorden, Patchouliöl, Cedern- und Sandelholzriechstoffen, interessante Effekte. Blumigen Herz(Mittel)noten verleihen die Verbindungen Eleganz und Strahlungskraft. Einige Beispiele für besonders gut harmonierende Stoffklassen sind:

- Naturprodukte, wie Eichenmoos Absolue, Geraniumöl, Jasmin Absolue, Patchouliöl, Rosenöl, Sandelholzöl, Vetiverol und Ylang-Ylang-Öl, etc.
- Alkohole, wie Citronellol, Ebanol®, Geraniol, Linalool, Phenylethylalkohol und Sandalore®, etc.
- Aldehyde und Ketone, wie Florozone® (3-(4-Ethylphenyl)-2,2-dimethyl-propional), Hydroxycitronellal, Iso-E-Super® (1,2,3,4,5,6,7,8-Octahydro-2,3,8,8-tetramethyl-2-octanaphthalin), Isoraldein®, Maltol, Methylcedrylketon, Methyljonon und Vanillin, etc.
- Ether und Acetale, wie Ambrox, Geranylmethylether, Rosenoxid und Spirambrene® (2',2',3,7,7-Pentamethylspiro[bicyclo[4.1.0]heptan-2,5'- [1,3]dioxan]), etc.
- Ester und Lactone, wie Berryflor®, g-Decalacton und g-Undecalacton, etc.

Ihre Vielseitigkeit ermöglicht einen breiten Einsatz der Thiamakrolide **I** nicht nur in den süssen orientalischen Kreationen, sondern auch in den Duftrichtungen 'Fougère', 'Chypre' und 'Floral'. Durch die niedrigen Schwellenwerte und die gute Haftfestigkeit werden neben der Luxusparfümerie auch Kompositionen für kosmetische Produkte, Waschmittel und ähnliche Massenerzeugnisse erschlossen.

Die Verbindungen der Formel **I** lassen sich in weiten Grenzen einsetzen, die beispielsweise von ca. 0.1 (Detergenzien) - ca. 40 Gew.-% (alkoholische Lösungen) in Kompositionen reichen können, ohne dass diese Werte jdoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen ca. 3 und ca. 20%. Die mit **I** hergestellten Kompositionen lassen sich für alle Arten von parfümistischen Verbrauchsgütern einsetzen (Eaux de Cologne, Euax de Toilette, Extraits, Lotionen, Crèmes, Shampoos, Seifen, Salben, Puder, Desodorantien, Detergenzien, etc.).

Die Verbindungen **I** können demgemäss bei der Herstellung von Kompositionen und -wie obige Zusammenstellung zeigt- unter Verwendung einer breiten Palette bekannter Riechstoffe bzw. Riechstoffgemische verwendet werden. Bei der Herstellung solcher Kompositionen können die oben angeführten Riechstoffe bzw. Riechstoffgemische nach (dem Parfümeur bekannter) Art und Weise verwendet werden, wie z. B. aus W. A. Poucher, Perfumes, Cosmetics, Soaps, 2. Bd., 7. Aufl., Chapman und Hall, London 1974, hervorgehend.

Die Herstellung dieser neuen Thismakrotide **I** wäre nun nach dem bekannten Verfahren [Chemische Fabriek Naarden, Niederländisches Patent No. 96391, 29 Juni, 1957] sehr aufwendig, da die dafür benötigten Ausgangsverbindungen (w-Alkenole) schwer zugänglich, nicht kommerziell erhältlich und sehr kostenintensiv sind. Dasselbe gilt für die übrigen Verfahren des Standes der Technik [V. N. Belov, N. P. Solov'eva, T. A. Rudol'fi, I. A. Voronina, *J. Org. Chem. USSR* (*Engl. Transl.*) **1965**, *1,* 539; H. Matsuyama, T. Nakamura, N. Kamigata, *J. Org. Chem.* **1989**, *54,* 5218].

Daher wird die Synthese der Substanzen **I** erfindungsgemäß durch einen neuen Zugang gelöst, der von aliphatischen Diolen sowie Lactonen **6** als gut zugänglichen Ausgangsmaterialien ausgeht.

Der Zugang zu den Thiamakroliden ist weiter unten schematisch dargestellt: Er beginnt mit einer Ringöffnung-Veresterung-Tandemreaktion der Lactone **6** in an sich bekannter Weise mit Trimethylsilyliodid und einem Alkohol R 'OH mit 1-6 Kohlenstoffatomen [M. Kolb, J. Barth, *Synth. Commun.* **1981**, *11*. 763], wobei Trimethylsilyliodid auch *in situ* generiert werden kann [z.B. G. A. Olah, S. C. Narrang, G. F. Salem, B. G. B. Gupta, *Synthesis* **1981,** 142].

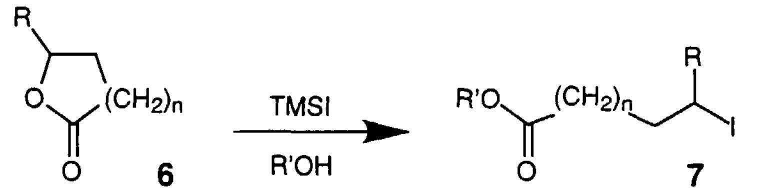


**6** (n=1–10, R= H, $C_1$–$C_4$ Alkyl)

**7** (R'= $C_1$–$C_6$ Alkyl, Aryl z. B. Phenyl, Benzyl)

Die Iodester **7** werden dann erfindungsgemäss, zweckmässigerweise in einer neuen Eintopfreaktion, direkt zu w-Hydroxy-(n+3)-thiacarbonsäuren **11** umgesetzt. Anders als bei ähnlichen Reaktionen des Standes der Technik [S. Fujisaki, I. Fujiwara, Y. Norisue, S. Kajigaeshi, *Bull. Chem. Soc. Jpn.* **1985,** *58,* 2429; Y. Takeuchi, K. Sakagawa, M. Kubo, M. Yamato, *Chem. Pharm. Bull.* **1986**, *34*, 1323], die nicht zum Produkt **10** führen, werden bei der erfindungsgemässen Reaktion das Tetramethylthiouroniumsalz **8** und das Monoalkoholat **9** aus dem entspechenden Diol zweckmässigerweise getrennt hergestellt und dann bei höheren Temperaturen, gewöhnlich unter Rückfluß in einem aprotischen Lösungsmittel, insbesondere in Acetonitril, miteinander umgesetzt.

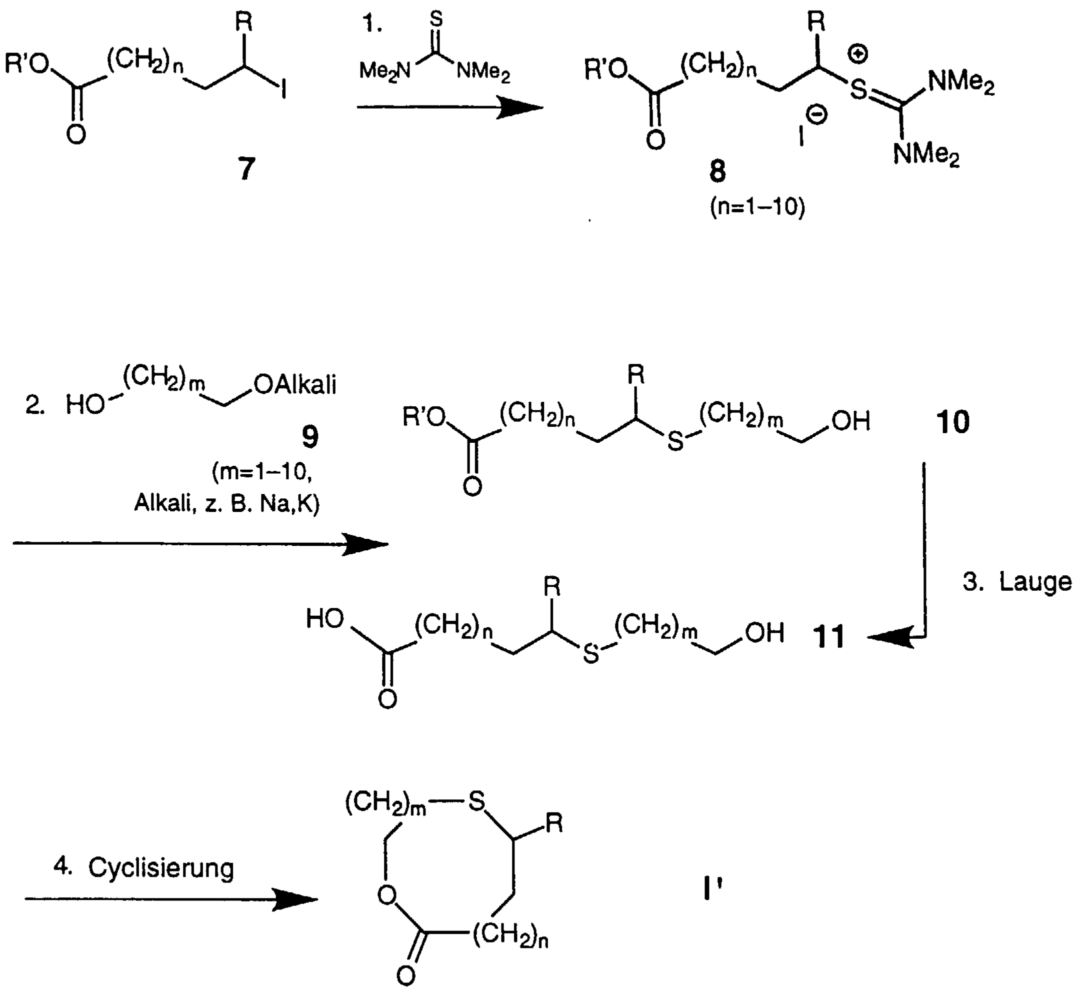


m + n=8–13

Die Cyclisierung der nach üblicher basischer Verseifung der Reaktionsprodukte **10** vorliegenden w-Hydroxy-(n+3)-thiacarbonsäuren **11** zu Thiamakroliden **I'** gelingt ohne Zwischenreinigung der Hydroxysäuren. Sie geschieht vorzugsweise durch Erhitzen von **11** mit einem Schleppmittel in Anwesenheit einer Base als Katalysator, z.B. in Anlehnung an das Verfahren von Collaud [Givaudan, U.S. Patent 2234551]. Als Schleppmittel geeignet sind insbesondere mono- oder polyvalente Alkohole, beispielsweise schwerer flüchtige Alkohole wie Glycerin oder Ethylenglykol, etc. Als Basen kommen insbesondere starke Basen wie Kalium- oder Natriummethylat in Frage. Geeignete Temperaturen sind Rückflusstemperaturen unter vermindertem Druck, die im Bereich von 100 °C bis ca. 200°C liegen.

Die Stereochemie der Verbindungen der Formeln **I** und **I'** wird durch den Rest (R) der eingesetzten Lactone **6** bestimmt. Damit lassen sich folglich enantiomeren-angereicherte und enantiomerenreine alkylsubstituierte Thiamakrolide herstellen. Die vorliegende Formeln **I** und **I'** umfassen demgemäss alle möglichen Stereoisomeren.

Bei den als Moschusriechstoffe geeigneten makrocyclischen Verbindungen **I'** mit R = $C_1$-$C_4$-Alkyl sollte die Gesamtsumme der Kohlenstoffatome zweckmässigerweise n+m+R = 14-17 C-Atome betragen.

Die folgenden Beispiele erläutern die Erfindung:

**Beispiel 1: 7-Thia -15-pentadecanolid (2)**

6-*Iodcapronsäureethylester* (**7**, n=3, R=H, R'=Et): Zu einer Lösung von 25 ml (0.24 mol) e-Caprolacton und 35 ml (0.62 mol) Ethanol in 200 ml Dichlormethan läßt man unter Argon bei 0°C 50 ml (0.37 mol) Trimethylsilyliodid zutropfen. Nach 1 stdg. Rühren bei Raumtemp. wird das Reaktionsgemisch auf 100 ml Wasser gegossen. Die organische Phase wird mit je 100 ml Wasser und ges. Natriumhydrogencarbonat-Lösung gewaschen, über Natriumsulfat getrocknet und am Rotationsverdampfer zur Trockne eingeengt. Nach Flashchromatographie (*n*-Pentan-MTBE, 9:1, h$R_f$ = 74) an Kieselgel erhält man 30 g (46%) 6-Iodcapronsäureethylester als farblose Flüssigkeit.

*15-Hydroxy-7-thiapentadecansäure* (**9**, n=3, m=7, R=H): Eine Lösung von 14 g (0.11 mol) Tetramethylthioharnstoff und 28 g (0.10 mol) 6-Iodcapronsäureethylester in 220 ml trockenem Acetonitril wird 2h unter Rückfluß erhitzt. In einem weiteren Reaktionsgefäß löst man unter Argon 23 g (0.16 mol) 1,8-Octandiol in 650 ml trockenem Acetonitril, versetzt die Lösung mit 4.5 g (0.11 mol) einer 60 proz. Natriumhydrid-Suspension in Mineralöl und erhitzt 45 min unter Rückfluß. Man läßt beide Ansätze auf 50°C abkühlen und vereinigt sie darauf vorsichtig innerhalb von 30 min. Die vereinigten Reaktionsgemische werden 14 h zum Rückfluß erhitzt, anschließend 3 h bei Raumtemp. mit 60 ml 10 proz. Kalilauge verseift, am Rotationsverdampfer aufkonzentriert und dann auf 1l Wasser gegeben. Man extrahiert zweimal mit je 500 ml MTBE und wäscht die organischen Extrakte dreimal mit je 50 ml 2N Natronlauge. Die wäßrige Phase wird mit konz. Phosphorsäure angesäuert und dreimal mit je 500 ml MTBE extrahiert. Nach Trocknen der vereinigten organischen Extrakte über Magnesiumsulfat und Entfernen des Lösungsmittels am Rotationsverdampfer erhält man 22 g rohe 15-Hydroxy-7-thiapentadecansäure als gelblichen wachsartigen Feststoff. Eine analytische Probe erhält man nach Flashchromatographie (MTBE / *n*-Pentan, 2:1, h$R_f$ = 33) an Kieselgel.

IR(Film): $\nu$ = 1691 $cm^{-1}$ ($\nu$ C=O), 1064 $cm^{-1}$ ($\nu$ C-OH), 1284/1267 $cm^{-1}$($\nu$ CO-O), 3423/3362 $cm^{-1}$ ($\nu$ O-H), 1198 $cm^{-1}$ ($\delta$ O-H). - $^1$H-NMR ($CDCl_3$): $\delta$ = 1.31-1.49 (m, 10H, 4-,10-$H_2$-13-$H_2$), 1.54-1.71 (m, 8H, 2-,5-,9-,14-$H_2$), 2.36 (t, *J* = 7.6 Hz, 2H, 2-$H_2$), 2.50 / 2.51 (t, *J* = 7.2 Hz, 4H, 6-,8-$H_2$), 3.65 (t, *J* = 6.6 Hz, 2H, 15-$H_2$). - $^{13}$C-NMR ($CDCl_3$): $\delta$ = 24.18 (t, C-3), 25.46 (t, C-13), 28.12 / 28.61 / 28.98 (t, C-4,-10,-11), 29.08 / 29.13 / 29.44 (t, C-5,-9,-12), 31.68 / 31.92 (t, C-6,-8), 32.26 (t, C-14), 33.78 (t, C-2), 62.57 (t, C-15), 178.59 (s, C-1). - MS (EI): m/z (%) = 143 (100) [$C_8H_{15}S^{\oplus}$], 161 (8) [$C_8H_{17}OS^{\oplus}$], 175 (4) [$C_9H_{19}OS^{\oplus}$], 229 (5) [$C_{12}H_{21}O_2S^{\oplus}$]; 276 (22) [$C_{14}H_{28}O_3S^{\oplus}$].

*7-Thia-15-pentadecanolid* (**2**): Man versetzt 22 g rohe 15-Hydroxy-7-thiapentadecansäure (**9,** n=3, m=7, R=H) mit 0.45 ml (4.0 mmol) 50 proz. Kalilauge und destilliert bei 180°C/20-23 mbar während 1 h Nebenprodukte ab. Darauf wird das Reaktionsgefäß mit Kühler und Abscheider ausgerüstet und 1.2 g (17 mmol) Kaliummethylat in 300 ml wasserfreiem Glycerin zum Reaktionsgemisch zugegeben. Man erhitzt 2 d bei 153°C/4-6 mbar zum Rückfluß über den Abscheider, wobei nach 14h erneut 1.2 g (17 mmol) Kaliummethylat zugegeben werden. Das abgeschiedene Glycerin wird auf 800 ml Wasser gegossen und das Rohprodukt zweimal mit je 500 ml MTBE und einmal mit 500 ml *n*-Pentan extrahiert. Die vereinigten organischen Extrakte werden über Magnesiumsulfat getrocknet und am Rotationsverdampfer zur Trockne eingeengt. Nach Flashchromatographie (*n*-Pentane/MTBE, 19:1, h$R_f$ = 46) an Kieselgel erhält man 2.9 g (11% Gesamtausbeute) 7-Thia-15-pentadecanolid als farblosen wachsartigen Feststoff mit einem *intensiven, linearen Moschusgeruch begleitet von grün-mosigen Akzenten* bei einem Schwellenwert von 0.2 ng/l Luft.

IR (Film): $\nu$= 1732 $cm^{-1}$ ($\nu$ C=O), 1247 $cm^{-1}$ ($\nu$ C-CO-O), 1124 $cm^{-1}$ ($\nu$ C-O-C). - $^1$H-NMR ($CDCl_3$): $\delta$ = 1.26-1.50 (m, 10H, 4-,10-$H_2$-13-$H_2$), 1.56-1.77 (m, 8H, 3-,5-,9-,14-$H_2$), 2.35 (t, *J* = 6.8 Hz, 2H, 2-$H_2$), 2.52 / 2.53 (t, *J* = 6.8 Hz, 4H, 6-,8-$H_2$), 4.15 (t, *J* = 5.2 Hz, 2H, 15-$H_2$). - $^{13}$C-NMR ($CDCl_3$): $\delta$ = 24. 68 (t, C-3), 25.16 (t, C-13), 26.11 (t, C-4), 27.12 / 27. 25 / 27.58 / 27. 73 (t, C-10,-11,-12,-14), 28.32 / 28.36 (t, C-5,-9), 30.66 / 30.92 (t, C-6,-8), 34.36 (t, C-2), 64.01 (t, C-15), 173.65 (s, C-1). - MS (EI): m/z (%) = 143 (100) [$c_8H_{15}S^{\oplus}$], 157 (7) [$C_9H_{17}S^{\oplus}$], 229 (7) [$C_{12}H_{21}O_2S^{\oplus}$], 258 (74) [$C_{14}H_{26}O_2S^{\oplus}$].

**Beispiel 2: 4-Methyl-5-thia-15-pentadecanolid (I, n=1, m=9, R=Me)**

*4-Iodvaleriansäureethylester* (**7**, n=1, R=Me, R'=Et): Analog zur Synthese des 6-Iodcapronsäureethylesters (Beispiel 1) werden 67 ml (0.96 mol) g-Valerolacton mit 140 ml (2.4 mol) Ethanol und 200 ml (1.4 mol) Trimethylsilyliodid umgesetzt. Nach üblicher Aufarbeitung und Flashchromatographie (*n*-Pentane/MTBE, 9:1, h$R_f$= 63) erhalt man 94 g (73%) 4-Iodvaleriansäureethylester als farblose Flüssigkeit.

*4-Methyl-5-thia-15-pentadecanolid* (**I**, n=1, m=9, R=Me): Analog zur Synthese des 7-Thia-15-pentadecanolids (Beispiel 1) über die 15-Hydroxy-7thiapentadecansäure werden 15 g (0.12 mol) Tetramethylthioharnstoff mit 25 g (0.96 mol) 4-Iodvaleriansäureethylester in 200 ml trockenem Acetonitril und parallel 20 g (0.12 mol) Decandiol in 640 ml trokkenem Acetonitril mit 5.1 g (0.12 mol) 60 proz. Natriumhydrid-Suspension in Mineralöl umgesetzt, die Reaktionsprodukte wie beschrieben vereinigt und 20 h unter Rückfluß erhitzt. Nach der üblichen Aufarbeitung erhält man 19 g rohe 15-Hydroxy-4-methyl-5-thiapentadecansäure, die mit 0.07 ml (0.62 mmol) 50 proz. Kalilauge polymerisiert und mit zweimal 0.9 g (13 mmol) Kaliummethylat in 300 ml Glycerin wie unter Beispiel 1 beschrieben depolymerisiert wird. Nach üblicher Aufarbeitung und Flashchromatographie erhält man 3.6 g (14% Gesamtausbeute) 4-Methyl-5-thia-15-pentadecanolid als farblosen wachsartigen Feststoff mit einem *süß-moschusartigem Geruch mit grünen und metallischen Nuancen* bei einem Schwellenwert von 1.8 ng/L Luft.

IR (Film): $\nu$ = 1734 $cm^{-1}$ ($\nu$ C=O), 1163/ 1194 $cm^{-1}$ ($\nu$ C-O), 1268 $cm^{-1}$ ($\nu$ C-CO-O). - $^1$H-NMR ($CDCl_3$): $\delta$ = 1.28 (d, *J* = 6.8 Hz, 3H, 4-Me), 1.31-1.65 (m, 16H, 7-$H_2$-13-$H_2$), 1.79 (dddd, *J* = 7.2, 6.8, 6.4 und 1.0 Hz, 1H, 3-$H_b$), 1.79

(dddd, *J* = 7.2, 6.8, 6.4 und 1.0 Hz, 1H, 3-$H_b$), 1.89 (dddd, *J* = 7.2, 6.8, 6.8 und 6.4 Hz, 1H, 3-$H_a$), 2.41 (ddd, *J* = 15.6, 6.4 und 6.4 Hz, 1H, 2-$H_b$), 2.49 (ddd, *J* = 12.8, 6.8 und 6.8 Hz, 1H, 6-$H_b$), 2.55 (ddd, *J* = 12.8, 6.8 und 6. 8 Hz, 1H, 6-$H_a$), 2.65 (ddd, *J* = 15.6, 7.2 und 7.2 Hz, 1H, 2-$H_a$), 2. 80 (qdd, *J* = 6.8, 6.8 und 1.0 Hz, 1H, 4-H), 4.06 (ddd, *J* = 10.8, 6.4 und 3.6 Hz, 1H, 15-$H_b$), 4. 23 (ddd, *J* = 10.8, 8.0 und 3.6 Hz, 1H, 15-$H_a$). - $^{13}$C-NMR ($CDCl_3$): $\delta$ = 21.43 (q, 4-Me), 24.25 (t, C-3), 26.10 / 26.11 / 26.20 (t, C-8,-13,-14), 26.69 / 27.01 / 27.89 / 28.11 (t, C-9,-10,-11,-12), 29.17 (t, C-7), 31.43 / 31.56 (t, C-2,-6), 38.87 (d, C-4), 63.79 (t, C-15), 173.53 (s, C-1). - MS (EI) m/z (%) = 87 (100) [$C_4H_7S^{\oplus}$], 101 (97) [$C_5H_9S^{\oplus}$], 114 (77) [$C_6H_{10}S^{\oplus}$], 132 (35) [$C_7H_{16}S^{\oplus}$], 171 (23) [$C_{10}H_{19}S^{\oplus}$], 257 (1) [$C_{14}H_{25}O_2S^{\oplus}$], 272 (16) [$C_{15}H_{28}O_2S^{\oplus}$].

Die folgenden Verbindungen sind in entsprechender Weise zugänglich. Von ihnen sind daher nur die spektroskopischen Daten aufgeführt:

**Beispiel 3: 6-Thia-16-hexadecanolid (1)**

*Intensiver Moschusgeruch mit pudrig-trockenem Effekt* bei einem Schwellenwert von 0.1 ng/l Luft. - IR (Film): $\nu$ = 1734 $cm^{-1}$ ($\nu$ C=O), 1256 $cm^{-1}$ ($\nu$ C-CO-O), 1181 / 1128 $cm^{-1}$ ($\nu$ C-O). - $^1$H-NMR ($CDCl_3$): $\delta$ = 1.32-1.46 (m, 12H, 9-$H_2$-14-$H_2$), 1.56-1.67 (m, 6H, 3-,4-,8-$H_2$), 1.72-1.79 (m, 2H, 15-$H_2$), 2.35 (t, *J* = 7.2 Hz, 2H, 2-$H_2$), 2.51 / 2.52 (2t, *J* = 7.2 Hz, 4H, 5-,7-$H_2$), 4.15 (dd, *J* = 5.6 und 5.6 Hz, 2H, 16-$H_2$). - $^{13}$C-NMR ($CDCl_3$): $\delta$ = 24.32 (t, C-3), 25.06 (t, C-14), 26.75 / 26.92 / 26.92 (t, C-10,-11,-12), 27.34 / 27.64 (t, C-9,-13), 28.32 / 28.46 (t, C-4,-8), 29.79 (t, C-15), 31.61 / 31.93 (t, C-5,-7), 34.28 (t, C-2), 64.05 (t, C-16), 173.43 (s, C-1). - MS (EI) m/z (%) = 87 (100) [$C_4H_7S^{\oplus}$], 101 (61) [$C_5H_9S^{\oplus}$], 117 (27) [$C_6H_{13}S^{\oplus}$], 171 (31) [$C_{10}H_{19}S^{\oplus}$], 272 (18) [$C_{15}H_{28}O_2S^{\oplus}$].

**Beispiel 4: 11-Thia-15-pentadecanolid (3)**

*Intensiver Moschusgeruch mit einer Note nach frisch gebügelter Wäsche und einem eigentlich für Nitromoschuskörper charakteristischen seifigwachsigen Akzent* bei einem Schwellenwert von 0.2 ng/l Luft. - IR (Film): $\nu$ = 1734 $cm^{-1}$ ($\nu$ C=O), 1245 $cm^{-1}$ ($\nu$ C-CO-O), 1164 $cm^{-1}$ ($\nu$ C-O). - $^1$H-NMR ($CDCl_3$): $\delta$ = 1.34-1.47 (m, 10H, 4-$H_2$-8-$H_2$), 1.58-1.75 (m, 8H, 3-,9-,13-,14-$H_2$), 2.34 (t, *J* = 7.2 Hz, 2H, 2-$H_2$), 2.51 / 2.52 (2t, *J* = 3.6 Hz, 4H, 10-,12-$H_2$), 4.14 (t, *J* = 4.8 Hz, 2H, 15-$H_2$). - $^{13}$C-NMR ($CDCl_3$): $\delta$ = 24.65 (t, C-3), 26.14 / 26.16 (t, C-13,-14), 26.65 / 26.87 (t, C-4,-8), 27.18 / 27.59 / 27.78 / 28.02 (t, C-5,-6,-7,-9), 30.48 / 30.86 (t, C-10,-12), 34.17 (t, C-2), 63.64 (t, C-15), 173.79 (t, C-1). - MS (EI) m/z (%) = 88 (100) [$C_4H_8S^{\oplus}$], 101 (14) [$C_5H_9S^{\oplus}$], 115 (6) [$C_6H_{11}S^{\oplus}$], 133 (6) [$C_7H_{17}S^{\oplus}$], 151 (7) [$C_9H_{11}O_2^{\oplus}$], 199 (7) [$C_{11}H_{19}OS^{\oplus}$], 217 (6) [$C_{11}H_{21}O_2S^{\oplus}$], 258 (10) [$C_{14}H_{26}O_2S^{\oplus}$].

**Beispiel 5: 6-Thia-14-tetradecanolid (4)**

*Süsse Moschusnote mit grün-erdigem Einschlag* bei einem Schwellenwert von 0.3 ng/l Luft. - IR (Film): $\nu$ = 1730 $cm^{-1}$ ($\nu$ C=O), 1261 / 1221 $cm^{-1}$ ($\nu$ C-CO-O), 1131 / 1156 $cm^{-1}$ ($\nu$ C-O). - $^1$H-NMR ($CDCl_3$): $\delta$ = 1.38-1.56 (m, 10H, 3-$H_2$,9-$H_2$-12-$H_2$), 1.63-1.70 (m, 4H, 4-,8-$H_2$), 1.73-1.78 (m, 2H, 13-$H_2$), 2.38 (t, *J* = 6.4 Hz, 2H, 2-$H_2$), 2.50 / 2.52 (2t, *J* = 7.6 / 6.4 Hz, 4H, 5-,7-$H_2$), 4.16 (dd, *J* = 5.2 und 4.0 Hz, 2H, 14-$H_2$). - $^{13}$C-NMR ($CDCl_3$): $\delta$ = 24.53 / 24.61 (t, C-3,-12), 25.78 (t, C-13), 26.35 / 26.83 (t, C-10,-11), 28.13 / 28.16 (t, C-4,-9), 30.51 (t, C-8), 31.85 / 32.86 (t, C-5,-7), 34.20 (t, C-2), 63.87 (t, C-14), 173.53 (s, C-1). - MS (EI) m/z (%) = 87 (100) [$C_4H_7S^{\oplus}$], 101 (55) [$C_5H_9S^{\oplus}$], 117 (32) [$C_6H_{13}S^{\oplus}$], 143 (51) [$C_8H_{15}S^{\oplus}$], 159 (4) [$C_8H_{15}OS^{\oplus}$], 215 (2) [$C_{12}H_{24}OS^{\oplus}$], 244 (21) [$C_{13}H_{24}O_2S^{\oplus}$].

**Beispiel 6:** Frisch-würziger Holz-Akkord, zur Verwendung in Herren-Parfüms und Körperpflegeprodukten geeignet

| | Gewichtsteile |
|---|---|
| 7-THIA-15-PENTADECANOLID (**2**) | 50 |
| BENZYLACETAT EXTRA | 30 |
| GERANYLACETAT REIN | 50 |
| 2-PENTYLOXYGLYKOLSÄUREALLYLESTER | 3 |
| METHYL ANTHRANILAT | 1 |
| BASILIKUMÖL | 10 |
| BERGAMOTTÖL | 200 |
| CARBITOL | 20 |
| METHYL-ALPHA-IONON | 50 |
| ZITRONENÖL ARGENTIN. | 100 |
| CUMARIN KRIST. | 20 |
| DIHYDROMYRCENOL | 100 |
| ESTRAGONÖL | 5 |
| EVERNYL | 3 |
| NELKENBLÜTENÖL | 15 |
| METHYLDIHYDROJASMONAT | 50 |
| ISO E SUPER | 50 |
| ISOEUGENOL | 3 |

| | |
|---|---|
| LAVANDINÖL | 100 |
| METHYLCEDRYLKETON | 60 |
| MUSKATNUSSÖL | 20 |
| PATCHOULIÖL | 30 |
| g-UNDECALACTON | 1 |
| PETITGRAINÖL PARAGUAY | 7 |
| SANDALORE | 20 |
| VANILLIN | 2 |
| | 1000 |

*7-Thia-15-pentadecanolid (**2**) verleiht der Komposition eine süße Moschusnote, zusätzliches Volumen und Geschmeidigkeit und unterstreicht den Körperpflege-Aspekt von Kosmetikartikeln.*

**Beispiel 7:** Frisches Blumen-Bouquet, zur Verwenduung in Seifen und Körperpflegeprodukten geeignet

| | Gewichtsteile |
|---|---|
| 11-THIA-15-PENTADECANOLID (**3**) | 50 |
| BENZYLACETAT EXTRA | 30 |
| L-BORNYLACETAT REIN | 6 |
| LINALYLACETAT SYNTH. | 80 |
| PARA-TERT.-BUTYLCYCLOHEXYLACETAT | 100 |
| VERDYL ACETAT | 15 |
| ALPHA-HEXYLZIMTALDEHYD | 130 |
| 2-PENTYLOXYGLYKOLSÄUREALLYLESTER | 3 |
| CEDERNHOLZÖL | 10 |
| DAMASCENON, 10 % DIETHYLPHTHALAT | 5 |
| DIHYDROMYRCENOL | 80 |
| 2,5-DIMETHYL-2-OCTEN-6-ON | 7 |
| FLORHYDRAL (3-(3-ISOPROPYLPHENYL)-BUTANAL) | 4 |
| GIVESCONE | 10 |
| METHYLDIHYDROJASMONAT | 40 |
| INDOLENE | 4 |
| ISORALDEINE 95 | 100 |
| CITRAL | 5 |
| LINALOOL SYNTH. | 200 |
| NECTARYL | 10 |
| OKOUMAL (2,4-DIMETHYL-2-(1,1,4,4-TETRAMETHYL-TETRALIN-6-YL)-1,3-DIOXOLAN) | 10 |
| TANGERINOL (6,10-DIMETHYL-5,9-UNDECADIEN-ESSIGSÄURE-2-YL ESTER) | 1 |
| ACETYLCEDREN | 100 |

*Das 11-Thia-15-pentadecanolid (**3**) verleiht der Komposition eine feine Moschusnote, gibt ihr Süsse und Volumen und betont den pflegenden Charakter von Kosmtikartikeln.*

Die obigen Fachbezeichnungen können z. B. anhand der Monographie Allured's Flavor and Fragrance Materials, 1996, Allured Publishing Company, Carol Stream, IL (USA), identifiziert werden.

**Patentansprüche**

**1.** Verbindungen der Formel

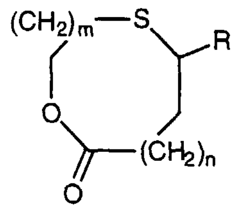

**I**

worin R=H und worin
m=9 und n=3, oder
m=7 und n=4, oder
m=3 und n=8, oder
m=7 und n=3 bedeuten.

**2.** 6-Thia-16-hexadecanolid.

**3.** 7-Thia-15-pentadecanolid.

**4.** 11-Thia-15-pentadecanolid.

**5.** 6-Thia-14-tetradecanolid.

**6.** Riechstoffkompositionen, gekennzeichnet durch einen Gehalt an einer Verbindung der Formel I gemäss Anspruch 1.

**7.** Verwendung einer Verbindung gemäss Anspruch 1 bis 5 als Riechstoff.

**8.** Verfahren zur Herstellung der Verbindungen der Formel

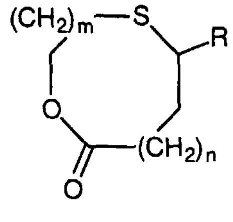

**I'**

worin R=H oder $C_1$-$C_4$ Alkyl
und m=1-10, n=1-10,
sowie n+m = 8-13 sind,

dadurch gekennzeichnet, dass man einen Iodester der Formel

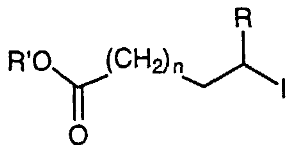
7

worin R' $C_{1-6}$-Alkyl oder Aryl bedeutet,
mit Tetramethylharnstoff zu einem Tetramethylthiouroniumsalz der Formel

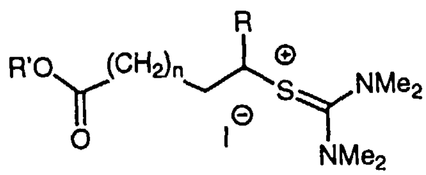
8

umsetzt, und dieses Salz **8** bei erhöhter Temperatur mit einem Monoalkoholat der Formel

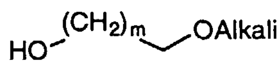
9

zu einer Verbindung

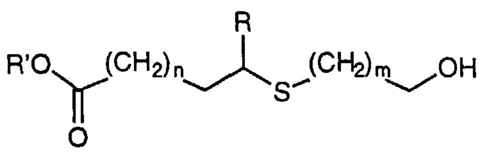
10

umsetzt, diese Verbindung **10** basisch zu einer Verbindung der Formel

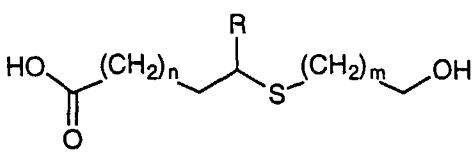
11

verseift, und die Verbindung **11** in an sich bekannter Weise zu einem Lacton der Formel **I'** cyclisiert.

**9.** Verfahren nach Anspruch 8, dadurch gekennzeichnet, dass man die Verbindung **11** durch Erhitzen mit einem Schleppmittel in Anwesenheit einer Base cyclisiert.